# EUROPEAN PATENT SPECIFICATION

(11) **EP 3 416 669 B1**
(45) Date of publication and mention of the grant of the patent: **28.05.2025**
(21) Application number: 17708179.1
(22) Date of filing: 16.02.2017
(51) Int. Cl.: A61K 38/10, C07K 14/47, C07K 7/08, A61K 38/00, A61P 1/04, A61P 11/00, A61P 11/04, A61P 13/00, A61P 15/00, A61P 17/00, A61P 17/02, A61P 21/00, A61P 29/00, A61P 31/02, A61P 31/04, A61P 43/00

(54) **MEANS AND METHODS FOR TREATING BACTERIAL INFECTIONS**
VERBINDUNGEN UND VERFAHREN ZUR BEHANDLUNG VON BAKTERIELLEN INFEKTIONEN
MOYENS ET PROCÉDÉS DE TRAITEMENT D'INFECTIONS BACTÉRIENNES

(30) Priority: 19.02.2016 EP 16156556
(43) Date of publication of application: 26.12.2018
(73) Proprietor: Brandenburg, Klaus, 22763 Hamburg (DE)
(72) Inventor: BRANDENBURG, Klaus, 22763 Hamburg (DE); HEINBOCKEL, Lena, 22765 Hamburg (DE); REILING, Norbert, 22926 Ahrensburg (DE); MARTINEZ DE TEJADA, Guillermo, 31003 Pamplona (ES); SCHÜRHOLZ, Tobias, 18055 Rostock (DE); MAUSS, Karl, 22145 Hamburg (DE); WEINDL, Günther, 10249 Berlin (DE)
(74) Representative: Vossius & Partner Patentanwälte Rechtsanwälte mbB
(86) International application number: PCT/EP2017/053487
(87) International publication number: WO 2017/140770

(56) References cited:
- EP-A1- 2 108 372
- WO-A1-00/12528
- T. GUTSMANN ET AL: "New Antiseptic Peptides To Protect against Endotoxin-Mediated Shock", ANTIMICROBIAL AGENTS AND CHEMOTHERAPY, vol. 54, no. 9, 1 September 2010 (2010-09-01), US, pages 3817 - 3824, XP055242250, ISSN: 0066-4804, DOI: 10.1128/AAC.00534-10
- YUE SUN ET AL: "Inhibitory Effects of Antimicrobial Peptides on Lipopolysaccharide-Induced Inflammation", MEDIATORS OF INFLAMMATION., vol. 31, no. 4, 1 January 2015 (2015-01-01), GB, pages 1066 - 8, XP055289657, ISSN: 0962-9351, DOI: 10.1016/j.bbrc.2008.02.046
- STREMPEL NIKOLA ET AL: "Potential Application of Antimicrobial Peptides in the Treatment of Bacterial Biofilm Infections", CURRENT PHARMACEUTICAL DESIGN, vol. 21, no. 1, 2015, pages 67 - 84, XP002760037, ISSN: 1381-6128
- MARR A K ET AL: "Antibacterial peptides for therapeutic use: obstacles and realistic outlook", CURRENT OPINION IN PHARMACOLOGY, ELSEVIER SCIENCE PUBLISHERS, NL, vol. 6, no. 5, 1 October 2006 (2006-10-01), pages 468 - 472, XP028058458, ISSN: 1471-4892, [retrieved on 20061001], DOI: 10.1016/J.COPH.2006.04.006
- KENSHI YAMASAKI ET AL: "Antimicrobial peptides in human skin disease", EUROPEAN JOURNAL OF DERMATOLOGY,, vol. 18, no. 1, 1 January 2008 (2008-01-01), pages 11 - 21, XP008155460, ISSN: 1167-1122, DOI: 10.1684/EJD.2008.0304
- ASHLEY L HILCHIE ET AL: "Immune modulation by multifaceted cationic host defense (antimicrobial) peptides", NATURE CHEMICAL BIOLOGY, vol. 9, no. 12, 14 November 2013 (2013-11-14), GB, pages 761 - 768, XP055289347, ISSN: 1552-4450, DOI: 10.1038/nchembio.1393

## Description

The present invention relates to a peptide consisting of or comprising 18 to 22 amino acids, wherein the amino acids in positions 1 to 22, counted from the N-terminus, are as follows (1) G; (2) C or lacking; (3) K; (4) K; (5) Y or F; (6) R; (7) R; (8) F; (9) K or R; (10) W; (11) K; (12) F; (13) K or R; (14) G; (15) K; (16) F, L or W; (17) F or W; (18) F, L or W; (19) W or F; (20) C or lacking; (21) F or G or lacking; (22) G or lacking
for use in a method of
(a) treating, ameliorating or preventing a bacterial infection of the skin;
(b) treating, ameliorating or preventing a bacterial wound infection; and/or
(c) promoting wound healing.

In this specification, a number of documents including patent applications and manufacturer's manuals is cited.

Severe bacterial infections are an ever-increasing threat worldwide. This is aggravated by the continued appearance of multi-resistant bacteria on the one hand and a lack of suitable novel antibiotic agents on the other hand. Owing to an ever-increasing number of elderly patients, the number of bacterial infections which are difficult to treat increases at a yet faster pace. For example, in Germany there are about 150,000 infections with MRSA (methicillin-resistant *Staphylococcus aureus*), about 10% of which are lethal owing to bacterial sepsis (according to Deutsche Sepsis-Gesellschaft, Infection, 41, Suppl. 1, Weimar Sepsis Update 2013). Experts estimate that about 50,000 patients per year die in German hospitals owing to an infection with resistant bacteria. Globally, the annual number of lethal cases of sepsis is estimated to be between 9 and 10 million. Further infections such as *Mycobacterium tuberculosis* present a serious problem for public health systems owing to lengthy therapies and enormous costs. Yet about 1 million patients die per year from this infection. The continued appearance of resistant strains (such as MDR and XDR strains) further complicates the situation.

On the other hand, also non-systemic bacterial infections require increasingly lengthy treatments. Morbidity owing to such non-systemic bacterial infections keeps increasing. Examples include erysipelas, impetigo, folliculitis, boil and carbuncle. The latter mentioned infections are usually not life-threatening, yet they significantly impact quality of life. Other non-systemic bacterial infections presenting a particular challenge for public health systems are those which cause chronic inflammation. Examples include chronic obstructive pulmonary disease (COPD). In particular, *Haemophilus influenzae* is known to be involved in chronic inflammation accompanying COPD.

EP 2 271 356 and EP 2 108 372 describes antimicrobial peptides with antibacterial activity. These documents fail to suggest the excellent performance of peptides in the treatment of the specific medical indication which is the bacterial infection of badly healing wounds. Moreover, these documents entirely fail to suggest a synergism with regard to antibacterial activity when combining two or more peptides or combining one or more peptides with known antibiotics, typically small organic molecule antibiotics and instead reports additive effects.

Heinbockel et al. (Antimicrobial agents and chemotherapy 57, 1480-1487 (2013)) describes an antimicrobial peptide with high endotoxine neutralization capacity. In the hands of the authors of Heinbockel et al., the mentioned peptidic antibiotic failed to exhibit synergy when used together with small organic molecule antibiotics, even at the highest concentrations used.

Antimicrobial peptides for the treatment of wound infections and skin diseases have been described in, for example, Duplantier and van Hoek, Frontier in Immunology 4, 1-14 (2013), Kenshi and Richard, Eur. J. Dermatol. 18, 11-21 (2008) and Mangoni et al. (doi: 10.1111/exd.12929). The described peptides are fundamentally different in terms of structure from the agents of the present invention.

Gutsmann et al. (Antimicrobial agents and chemotherapy 54, 9, 3817-3824 (2010) describes the design of cationic amphiphilic peptides (synthetic antilipopolysaccharide (anti-LPS) peptides, SALPs) based on *Limulus* anti-LPS to neutralize bacterial endotoxins *in vitro* (human mononuclear cells) and *in vivo* (mouse). The described peptides are fundamentally different in terms of their function as a therapeutic strategy for clinical management of patients with septic shock compared to the agents of the present invention. Treatment, amelioration or prevention of a bacterial infection of the skin and/or promotion of wound healing is not described in this document.

Sun et al., (Mediators of Inflammation, (2015), Article ID: 167572), is a scientific review article which describes antimicrobial peptides (AMPs) in particular those neutralizing lipopolysaccharides and removing Gram-negative bacteria, as well as inhibition of inflammatory cytokine production to reduce inflammation. While the review describes the promotion of wound healing by AMPs, neither the treatment of bacterial infection of the skin nor the synergistic effect of an AMP with another peptide or compound as described in the present invention is described.

The document WO 00/12528 describes cationic peptides having antimicrobial activity, which differ in sequence from those described in the present disclosure. The peptides in this document are described to treat conditions resulting from a gram-negative bacterial infection and further use for accelerating wound healing in a subject, however, do not describe bacterial infections of the skin. The cationic nature of AMPs is not a general indication for a potential effectiveness in the promotion of wound healing, as there are also cationic peptides that do not exhibit a positive effect on the promotion of wound healing, therefore the AMPs of WO 00/12528 A1 do not suggest the effectiveness of the agents of the present invention in wound healing.

The technical problem underlying the present invention can be seen in the provision of improved means and methods for the treatment of bacterial infections, in particular infections by multi-resistant bacteria, including wound infections, skin infections and sepsis.

This technical problem has been solved by the subject-matter of the claims enclosed herewith.

The present disclosure relates to a peptide comprising or consisting of the sequence of SEQ ID NO:1 (GKKYRRFRWKFKGKLFLFG). The peptide consisting of this sequence is also referred to as peptide 19-4LF or Aspidasept II.

The term "peptide" generally describes linear molecular chains of amino acids containing up to 30 amino acids covalently linked by peptide bonds. The total number of amino acids comprised in the peptide may increase to preferably up to 30 if one or more amino acids are added to the sequence of SEQ ID NO: 1 at the N- and/or C-terminus. Said amino acids may or may not contribute to the functionality of the peptide. In other words, amino acid(s) added may or may not confer a distinct function to the peptide, be it its antimicrobial activity or another function.

The number of amino acids may further increase if the peptide of the disclosure is fused to another peptide or to a polypeptide. Fusion constructs are described in EP 2 271 356. Peptides may form oligomers consisting of at least two identical or different molecules. The corresponding higher order structures of such multimers are, correspondingly, termed homo- or heterodimers, homo- or heterotrimers etc.

The one-letter code abbreviations as used to identify amino acids throughout the present invention correspond to those commonly used for amino acids.

The peptide of the present disclosure can be produced synthetically.

Chemical synthesis of peptides is well known in the art. Solid phase synthesis is commonly used and various commercial synthesizers are available, for example automated synthesizers by Applied Biosystems Inc., Foster City, CA; Beckman; or MultiSyntech, Bochum, Germany. Solution phase synthetic methods may also be used. For example, peptide synthesis can be carried out using N-α-9-fluorenylethoxycarbonyl amino acids and a preloaded trityl resin or an aminomethylated polystyrene resin with a p-carboxytritylalcohol linker. Couplings can be performed in dimethylformamide using N-hydroxybenzotriazole and 2-(1H-benzotriazole-1-yl)-1,1,3,3-tetramethyluronium hexafluorophosphate. Commonly used side chain protecting groups are tert-butyl for N, Q, S and T; 2,2,4,6,7-pentametyldihydroxybenzofruan-5-sulfonyl for R; and butyloxycarbonyl for K. After synthesis, the peptides are deprotected and cleaved from the polymer support, e.g. by treatment with e.g. 92% trifluoracetic acid/ 4% triethylsilane/ 4% H₂O. The peptides can be precipitated by the addition of tertbutylether/pentane (8:2) and purified by reversed-phase HPLC. The peptides are commonly analyzed by matrix-associated laser desorption time-of-flight mass spectrometry. By using these standard techniques, naturally occurring amino acids may also be substituted with unnatural amino acids such as D-stereoisomers, and also with amino acids with side chains having different lengths or functionalities. Functional groups for conjugating to small molecules, label moieties, peptides, or proteins may be introduced into the molecule during chemical synthesis. In addition, small molecules and label moieties may be attached during the synthetic process. Preferably, introduction of the functional groups and conjugation to other molecules minimally affects the structure and function of the subject peptide.

The N- and C-terminus of the peptide as well as any amino acid comprised in the peptide apart from the terminal amino acids may be derivatized using conventional chemical synthetic methods. The peptides of the disclosure may contain an acyl group, preferably C₁ to C₄ acyl such as an acetyl group. Methods for acylating, and specifically for acetylating the free amino group at the N-terminus are well known in the art. For the C-terminus, the carboxyl group may be modified by esterification with alcohols, preferably C₁ to C₄ alkanols, or amidated to form - CONH₂ or CONHR, R preferably being C₁ to C₄ alkyl. Methods of esterification and amidation are well known in the art.

The peptide of the disclosure may also be produced semi-synthetically, for example by a combination of recombinant and synthetic production. In the case that fragments of the peptide are produced synthetically, the remaining part of the peptide would have to be produced otherwise, e.g., recombinantly, and then be linked to the fragment to form the peptide of the disclosure. Recombinant production is described in EP 2 271 356.

The specific sequence of SEQ ID NO: 1 is embraced by the genus of peptides disclosed in EP 2 271 356. This document, however, fails to individualize this specific sequence, nor have its particularly outstanding antibiotic properties been recognized.

The present disclosure provides a pharmaceutical composition comprising or consisting of a peptide in accordance with the disclosure.

In accordance with the present disclosure, the term "pharmaceutical composition" relates to a composition for administration to a patient, preferably a human patient. The pharmaceutical composition of the invention preferably comprises the peptide of the invention. It may, optionally, comprise further molecules capable of altering the characteristics of the peptide of the invention thereby, for example, stabilizing, modulating and/or activating its function. The composition may be in solid, liquid or gaseous form and may be, *inter alia,* in the form of (a) powder(s), (a) tablet(s), (a) solution(s) or (an) aerosol(s). The pharmaceutical composition of the present invention may, optionally and additionally, comprise a pharmaceutically acceptable carrier. By "pharmaceutically acceptable carrier" is meant a non-toxic solid, semi-solid or liquid filler, diluent, encapsulating material or formulation auxiliary or excipient of any type. Examples of suitable pharmaceutical carriers are well known in the art and include phosphate buffered saline solutions, water, emulsions, such as oil/water emulsions, various types of wetting agents, sterile solutions, organic solutions, organic solvents including DMSO. Compositions comprising such carriers can be formulated by well known conventional methods.

The pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently.

The therapeutically effective amount for a given situation will readily be determined by routine experimentation and is within the skills and judgment of the ordinary clinician or physician. Generally, the regimen as a regular administration of the pharmaceutical composition should be in the range of 1 µg to 5 g units per day. However, a more preferred dosage might be in the range of 0.01 mg to 100 mg, even more preferably 0.01 mg to 50 mg and most preferably 0.01 mg to 10 mg per day.

The pharmaceutical composition of the present invention may be administered systemically, topically or parenterally. The term "parenteral" as used herein refers to modes of administration which include intravenous, intramuscular, intraperitoneal, intrasternal, subcutaneous and intraarticular injection and infusion. Topical routes of administration include dermal, nasal and via inhalation.

In a first embodiment, the present invention provides a peptide consisting of or comprising 18 to 22 amino acids, wherein the amino acids in positions 1 to 22, counted from the N-terminus, are as follows (1) G; (2) C or lacking; (3) K; (4) K; (5) Y or F; (6) R; (7) R; (8) F; (9) K or R;; (10) W; (11) K; (12) F; (13) K or R; (14) G; (15) K; (16) F, L or W; (17) F or W; (18) F, L or W; (19) W or F; (20) C or lacking; (21) F or G or lacking; (22) G or lacking, for use in a method of (a) treating, ameliorating or preventing a bacterial infection of the skin; (b) treating, ameliorating or preventing a bacterial wound infection; and/or (c) promoting wound healing.

The definition of the peptide in accordance with the first embodiment embraces the specific peptide sequence in accordance with the first aspect. It is understood that, if not indicated explicitly to the contrary, peptides in accordance with any aspect of the present invention are linear and non-cyclic.

The present invention for the first time makes the above class of peptides available for the specific indications in accordance with items (a), (b) and (c), namely bacterial infections of the skin, bacterial wound infections and furthermore for the purpose of promoting wound healing. This is based on the present inventors' surprising finding that peptides in accordance with the third aspect, including the peptide of SEQ ID NO: 2 (as disclosed further below), allowed to combat bacterial infections of a badly closing wound where numerous attempts of established antibiotic therapy failed. Accordingly, not only is the prior art silent about usefulness of the peptides in accordance with the third aspect for the purposes in accordance with the third aspect, but furthermore are said peptides characterized by outstanding properties. In the view of the fact that any other antibiotic treatment of the mentioned badly healing wound failed, it has to be concluded that the peptides in accordance with the third aspect are in fact unique when compared to what has been previously available in terms of treatment options. Reference is made in particular to Example 5 in that respect.

The following preferred embodiments of the first embodiment relate to preferred peptides, preferred bacteria, preferred medical indications, and envisaged underlying mechanisms.

Accordingly, in a preferred embodiment, (a) said peptide (i) comprises or consists of the sequence of SEQ ID NO: 1; or (ii) comprises or consists of the sequence of SEQ ID NO: 2 (GCKKYRRFRWKFKGKFWFWG); and/or (b) said use is topical.

The peptide consisting of the sequence of SEQ ID NO: 2 is also known as peptide 19-2.5 or Aspidasept^{®}.

The term "topical" has its art-established meaning and refers to an administration of a peptide in accordance with the third aspect to the area affected by the bacterial infection and/or the area where wound healing is to be promoted and/or the proximity of the respective areas.

Suitable formulations for topical applications include ointments, solutions and sprays. Each of these formulations may comprise one or more pharmaceutically acceptable carriers, diluents or excipients. Compounds and compositions suitable as carriers, diluents and excipients are well known to the skilled person, described above, and available from a variety of manufacturers.

Furthermore, the formulations for topical application may comprise a deliver enhancer. Delivery enhancers are known in the art; see, e.g. WO 2011/064316. In a further preferred embodiment, said bacterial infection of the skin or said bacterial wound infection is an infection by Gram positive and/or Gram negative bacteria, preferably by one or more of *Staphylococcus* such as *Staphylococcus aureus* including MRSA, β-hemolytic *Streptococcus* including group A *Streptococcus* such as *Streptococcus pyogenes, Parvimonas* including *Parvimonas micra, Pseudomonas* such as *Pseudomonas aeruginosa* including MDRPA, *Haemophilus* including *Haemophilus influenza, Clostridium* including *Clostridium difficile, Enterococcus* including vancomycin-resistant *Enterococcus* (VRE), *Porphyromonas* including *Porphyromonas gingivalis, Propionibacterium* including *Propionibacterium acne* and *Acinetobacter* including *Acinetobacter baumannii.*

The peptides of the present invention are useful with respect to combating in particular multiresistant strains, independently of the kind of resistance mechanism, see Examples 1, 2 and 4.

In a further preferred embodiment, said bacterial infection of the skin or said bacterial wound infection is selected from erysipelas, impetigo, folliculitis, boil, cellulitis and carbuncle and/or is a nosocomial infection.

The above-mentioned specific indications are known in the art. In brief, erysipelas is an acute infection of the skin which predominantly affects the upper dermis and superficial lymphatics. Typical bacteria are group A *Streptococcus,* in particular *Streptococcus pyogenes.* Non-group A *Streptococci* may also be causative agents, but also rapidly growing mycobacteria, such as M. *fortuitum,* chelonae-abscessus group. For example, *Streptococcus agalactiae* is a non-group A *Streptococcus.* Cellulitis, which is also a bacterial infection, affects the inner layers of the skin, especially the dermis and the subcutaneous fat. A typical bacterium is *Staphylococcus aureus.* Of particular concern are the antibiotic resistant forms thereof such as methicillin-resistant *Staphylococcus aureus* (MRSA). Folliculitis is an infection of the hair follicles. Relevant bacterial species includes *Staphylococcus aureus* and *Pseudomonas aeruginosa.* Impetigo, in particular impetigo contagiosa, is a highly infectious disease of the skin which predominantly occurs in children and newborn. Typical bacteria are *Staphylococcus aureus* and *Streptococcus pyogenes.*

In terms of mechanism, it is envisaged that said peptide (a) inactivates the bacteria causing said infection; (b) binds and/or inactivates the toxins produced the bacteria causing said infection; and/or (c) acts anti-inflammatory.

Inactivation of bacteria includes a slowing down of bacterial growth, inhibition of bacterial growth, reduction of bacterial viability, partial killing of bacteria, and complete killing of bacteria.

The peptides in accordance with the invention are synthetic anti-lipopolysaccharide peptides (SALPs). Lipopolysaccharides (LPS) are comprised in the exterior membrane of Gram-negative bacteria. They function as antigens. Decay products thereof are released when bacteria die. These decay products are also known as endotoxins. Endotoxins contribute to the symptoms of a bacterial infection. The SALPs in accordance with the invention bind with high affinity to LPS and endotoxins. This significantly contributes to the beneficial effects of the peptides. In other words, the effect of endotoxins is neutralized by the peptides of the invention.

In a second embodiment, the present invention relates to a pharmaceutical composition comprising or consisting of a combination of (a) two or more peptides, each peptide consisting of or comprising 17 to 23 amino acids, wherein the amino acids in positions 1 to 23, counted from the N-terminus, are as follows (1) G, S or lacking; (2) C or lacking; (3) K or R; (4) K or R; (5) Y, W or F; (6) K or R; (7) K or R; (8) F, W or L; (9) K or R; (10) K or L or lacking; (11) W, L or F; (12) K or R; (13) F, Y or C; (14) K or R; (15) G or Q; (16) K or R; (17) F, L or W; (18) F or W; (19) F, L or W; (20) W or F; (21) C or lacking; (22) F or G or lacking; (23) G or lacking; or (b) one or more peptides, each peptide consisting of or comprising 17 to 23 amino acids, wherein the amino acids in positions 1 to 23, counted from the N-terminus, are as follows (1) G, S or lacking; (2) C or lacking; (3) K or R; (4) K or R; (5) Y, W or F; (6) K or R; (7) K or R; (8) F, W or L; (9) K or R; (10) K or L or lacking; (11) W, L or F; (12) K or R; (13) F, Y or C; (14) K or R; (15) G or Q; (16) K or R; (17) F, L or W; (18) F or W; (19) F, L or W; (20) W or F; (21) C or lacking; (22) F or G or lacking; (23) G or lacking, and one or more antibiotics selected from small organic molecule antibiotics such as ceftriaxone, oxacillin, amoxicillin, amikacin, ciprofloxacin, erythromycin, imipenem and tetracycline, and peptidic antibiotics such as daptomycin and vancomycin.

This embodiment of the invention relates to binary, ternary or higher order combinations of peptides or peptides with known antibiotics.

It is noted that certain peptides falling under the terms of the generic definition provided in part (a) of the second embodiment as well as the antibiotics recited in the second part of item (b) of the second embodiment are known. What was entirely unexpected, though, is the performance afforded by combinations as defined in accordance with the fourth aspect. The examples enclosed herewith contain evidence of very good performance for a plurality of combinations meeting the terms of the second embodiment. The difference between combinations and individual agents is not only astonishing in a quantitative sense as evidenced by art-established measures of synergistic effect, but also in qualitative terms. For example, while individual agents merely reduce bacterial growth, combinations in accordance with the fourth aspect are capable of entirely abolishing bacterial growth.

In a preferred embodiment of the second embodimentsaid pharmaceutical composition is a broad-spectrum antibiotic.

Antibacterial activity can be determined by any of the art-established measures. For example, for bacterial growth and suspension, photometric quantification may be used. This has been done, for example, in Example 2. Also, antibacterial activity may be determined via the number of colony forming units (CFU) in a nutrient.

The combinations in accordance with the invention are not only characterized by outstanding performance and a high degree of synergistic activity, but furthermore in that they are active against a large number of bacteria. In that sense, the combinations in accordance with the second embodiment are broad-spectrum antibiotics. As a consequence, the combinations in accordance with the second embodiment are the first choice, especially in those cases where diagnosis, e.g., determining which bacteria are responsible for a given infection, is cumbersome or takes too long. This applies, for example, to sepsis. The examples enclosed herewith contain evidence of activity of combinations in accordance with the invention against the plurality of highly problematic bacteria. In particular, the evidence relates to multi-resistant *E*. *coli* ESBL (extended spectrum beta lactamase) as shown in Example 1, multi-resistant *Staphylococcus aureus* (MRSA) as shown in Example 2, and multi-resistant *Pseudomonas aeruginosa* PS-4.

To assess the synergy between peptides, the Fractional Inhibitory Concentration (FIC) index of each combination was calculated according to the following formula: [(A)/MICA] + [(P)/MICP] = FICA + FICP = FIC index where MICA and MICP are the MICs of the two agents determined separately. The same applies *mutatis mutandis* to ternary or higher order combinations. Values below 1 indicate synergy, a value of 1 indicates additivity, and values greater than 1 indicate antagonism.

Surprisingly, it has been found that even sub-inhibitory concentrations of peptides in accordance with the present invention provide for a synergistic effect with art-established antibiotics such as gentamycin, levofloxacin or oxacillin. Without wishing to be bound by a specific theory, it is considered that said sub-inhibitory concentrations (as well as higher concentrations) induce a massive entry of antibiotics into the bacterial cells.

In a further preferred embodiment of the second embodiment, said combination is (a) a binary combination of (i) a peptide comprising or consisting of the sequence of SEQ ID NO: 1 and a peptide comprising or consisting of the sequence of SEQ ID NO: 2; (ii) a peptide comprising or consisting of the sequence of SEQ ID NO: 1 and an antibiotic as defined in accordance with the second embodiment; or (iii) a peptide comprising or consisting of the sequence of SEQ ID NO: 2 and an antibiotic as defined in accordance with the second embodiment; or (b) a ternary combination of a peptide comprising or consisting of the sequence of SEQ ID NO: 1, a peptide comprising or consisting of the sequence of SEQ ID NO: 2 and an antibiotic as defined in accordance with the second embodiment.

Among the antibiotics which may be combined with peptides in accordance with the present invention, ceftriaxone and oxacillin are particularly preferred.

The peptides comprising or consisting of the sequences of SEQ ID NOs: 1 and 2, respectively, are particularly preferred agents in accordance with the present invention.

In a further preferred case as part of the disclosure, (a) said pharmaceutical composition comprises a pharmaceutically acceptable carrier, diluent, or excipient; and/or (b) the recited pharmaceutically active agents are the only pharmaceutically active agents comprised in said pharmaceutical composition.

As common in the art, pharmaceutical compositions in accordance with the present invention may contain, in addition to pharmaceutically active agents, also pharmaceutically inactive agents including carriers, diluents and/or excipients. Exemplary carriers, diluents and excipients are described herein above.

Furthermore, it is preferred that no further pharmaceutically active agents beyond those explicitly recited are present in the pharmaceutical composition. Having said that, it is also envisaged that further, not explicitly mentioned pharmaceutically active agents are present. Among those further not explicitly mentioned agents, there is a preference for antibiotics.

In a third embodiment, the present invention provides a pharmaceutical composition of the second embodiment for use in (a) a method of treating, ameliorating or preventing one or more conditions selected from sepsis, bacterial infections of the respiratory tract, bacterial infections of the gastrointestinal tract, bacterial infections of the urogenital tract, necrotizing fasciitis, bacterial infections of burns, bacterial wound infections, and bacterial infections of the skin; or (b) a method of promoting wound healing.

As noted above, sepsis is a particularly preferred indication in accordance with the present invention. The combinations, owing to their high antibiotic activity against a broad spectrum of bacteria are the first choice where rapid action against the diseases required which otherwise would become uncontrollable. As such, combinations of the invention are useful in the treatment of systemic infections. Having said that, also non-systemic disorders are among the indications amenable to treatment.

With regard to promoting wound healing in accordance with above item (b), it is noted that independent of the presence of bacteria or their toxins, the peptides of the invention are able to stimulate metalloproteinases such as the ADAMs and are able to recruit growth factors like the epidermal growth factor EGF.

In preferred embodiments of the third embodiment, (a) the bacterial infection of the respiratory tract is tuberculosis, cystic fibrosis or COPD; (b) the bacterial infection of the gastrointestinal tract is Morbus Crohn; or (c) said condition is caused by Gram positive and/or Gram negative bacteria, especially by one or more of *Staphylococcus* such as *Staphylococcus aureus* including MRSA, *Mycobacterium* such as *Mycobacterium tuberculosis* including MDR and XDR strains, *Pseudomonas* such as *Pseudomonas aeruginosa* including MDRPA, *Enterococcus* including vancomycin-resistant *Enterococcus* (VRE), *Haemophilus* including *Haemophilus influenzae, E. coli* including ESBL, *Klebsiella* including *Klebsiella pneumonia,* β-hemolytic *Streptococcus* including group A *Streptococcus* such as *Streptococcus pyogenes,* and *Acinetobacter* including *Acinetobacter baumannii.*

The further implementations in accordance with the present dislosure as described below relate to the finding that individual peptides as well as combinations in accordance with the present invention are useful agents against biofilms. Biofilms are layers of bacteria on various types of surfaces, especially in hospitals and/or on devices which layers of bacteria are highly undesirable. The examples enclosed herewith contain evidence that agents in accordance with the present invention are capable of significantly reducing biofilms.

Accordingly, in a further embodiment, the present invention provides a method of preventing or reducing formation of a biofilm on a device for intracorporeal use or on a surface in a hospital and/or of removing of a biofilm from a device for intracorporeal use or from a surface in a hospital, wherein said device is not present in a human or animal body, said method comprising bringing said device or surface into contact with a pharmaceutical composition as defined above or a peptide as defined above.

The term "biofilm" is known in the art and refers to a mucus layer with microorganisms embedded therein. Biofilms are formed by microorganisms at surfaces and adhere to the surface. In other words, a biofilm may also be referred to as a multicellular surface bound aggregate. Microorganisms may comprise or consist of bacteria of one species or a plurality of species.

Prevention or reduction of biofilm formation and removal of a biofilm may, depending on the case, be a cosmetic or medical application. Any method of prevention, reduction or removal may comprise further measures such as scrubbing.

The above recited requirement that the intracorporeal device is not present in a human or animal body ensures that methods of treatment therapy of the human or animal body are not within the ambit of the respective aspect of the invention. To the extent methods of therapeutic treatment of the human or animal body are not excluded from patentability, this negative feature is dispensable.

In a further embodiment, the present invention provides a use of a pharmaceutical composition as defined above or a peptide as defined above for preventing or reducing formation of a biofilm on a device for intracorporeal use or on a surface in a hospital and/or for removing of a biofilm from a device for intracorporeal use or surface in a hospital, wherein said device is not present in a human or animal body.

In a further embodiment, the present disclosure provides an intracorporeal device or a surface in a hospital which is coated and/or loaded with a pharmaceutical composition as defined above or a peptide as defined above.

The term "coated" refers to a layer on the surface of the intracorporeal device or the surface in a hospital which comprises or consists of one or more peptides and/or pharmaceutical compositions according to the invention. Said layer may cover all or parts of the surface or intracorporeal device.

The term "loaded" refers to an intracorporeal device or surface, wherein said device or surface in its entirety or parts thereof are made of a material which comprises one or more peptides and/or pharmaceutical compositions as defined herein above.

In preferred case as part of the disclosure relating to the control of biofilms, (a) said device for intracorporeal use is selected from catheters, implants, endoscopes, drainages, contact lenses and hearing aids; or (b) said surface in a hospital is a fitting.

The Figures show:
- **Figure 1:**: Bacterial growth in presence and absence of agents of the invention.
- **Figure 2:**: Inactivation of a biofilm from *Pseudomonas aeruginosa* with GFP-labelled bacteria (GFP: Green-Fluorescent Protein). Living bacteria: white, inactivated bacteria: dark.
- **Figure 3:**: Peptide 19-2.5/Peptide 9-4LF/levofloxacin (A). Peptide 19-2.5/ Peptide 9-4LF/levofloxacin (B). The area under the curve (AUC) is a measure of the antimicrobial effectiveness of the drugs, with lowest values exhibiting the strongest effects.
- **Figure 4:**: Wound before and after daily treatment at t = 0, 3 months and 6 months.
- **Figure 5:**: Does dependent inhibition of *M*. *tuberculosis-induced* TNF formation of human peripheral blood mononuclear cells by Peptide 19.2.5. Supernatants of Isoniazid-treated *M. tuberculosis* bacteria (0,1 µg/ml; 72 h) were left untreated or incubated for 30 min with the peptides X, Y, Z at the concentrations indicated. Equal amounts were subsequently added to human PBMC (1×10⁶/ml) and incubated for further 24 h. The TNF formation was measured by ELISA (R&D Systems).
- **Figure 6:**: Aspidasept^{®} inhibits maturation and migration of MoDCs.
- **Figure 7:**: Aspidasept^{®} and Aspisasept II reduce IL-8 release in TLR2/6-activated keratinocytes.
- **Figure 8:**: Inhibition of biofilm formation.
- **Figure 9:**: Synergistic effect of the combination of levofloxacin with a peptide of the invention. Data were obtained from growth experiments in Mueller-Hinton (cation adjusted) with continuous shaking at 37°C using Bioscreen C, Labsystem, Helsinki, Finland. Concentrations were adjusted after checkerboard analysis, once Fractional Inhibitory Concentrations (FIC) were calculated. All cases resulted in a FIC index <0,5, indicating synergistic effect. Results show the average of three independent experiments.
- **Figure 10:**: Synergistic effect of the combination of the combination of gentamycin (A) and oxacillin (B), respectively, with a peptide of the invention.
- **Figure 11:**: Synergistic combination of peptides of the invention.

The Examples illustrate the invention.

### Example 1

### Growth of Escherichia coli ESBL (CUN E20) in the presence of combinations of ceftriaxon and peptides of the invention

The ability of the peptides to induce bacterial sensitization to antibiotics was determined by a standard checkerboard titration method in 96-well polystyrene microtiter plates [Eliopoulos GM, Moellering, RC: Antimicrobial combinations. In Antibiotics in Laboratory Medicine 4th edition. Edited by: Lorian V. Baltimore: The Williams and Wilkins Co; 1996:330-396]. For this purpose, serial dilutions of the two antimicrobial agents were mixed together in a microtiter plate so that each row contained a fixed amount of one agent and increasing amounts of the other. Inocula consisted of 1 × 10⁵ CFU/mL, approximately in Mueller-Hinton (MH) medium (Difco Laboratories, Sparks, MD, USA). To assess the synergy between peptides, the Fractional Inhibitory Concentration (FIC) index of each combination was calculated according to the following formula: [(A)/MICA] + [(P)/MICP] = FICA + FICP = FIC index, where MICA and MICP are the MICs of the two agents determined separately, and (A) and (P) are the MICs of the agents when determined in combination. A given peptide-peptide combination was considered as synergistic if its FIC index was ≤ 0.5. Peptides with a MIC higher than the maximum concentration tested (250 µg/mL) were arbitrarily assigned a MIC value of 500 µg/mL.

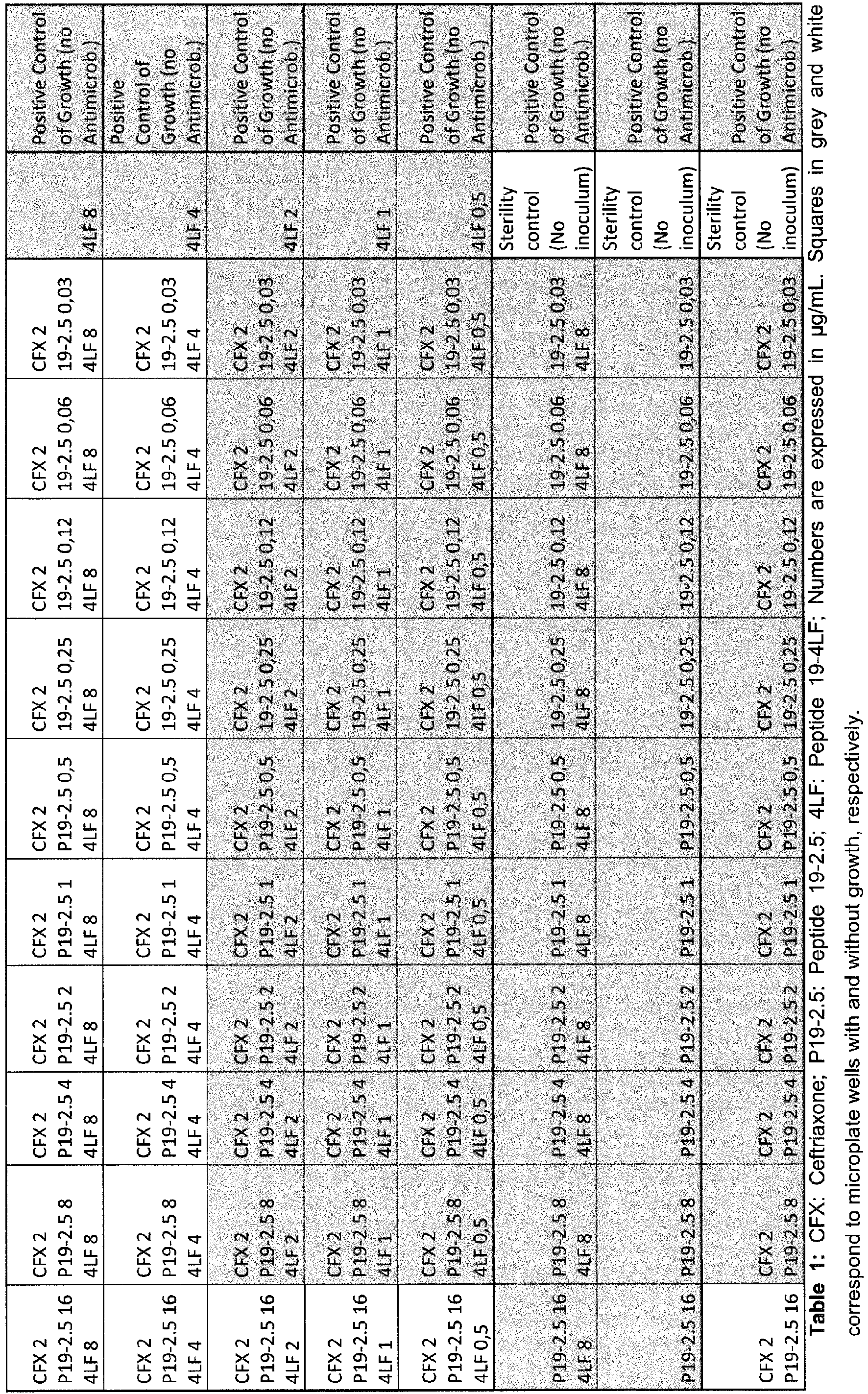

The MIC values for the individual agents are as follows: Peptide 19-4LF: 16 ug/ml; Peptide 19-2.5: 128 ug/ml; Ceftriaxon: 16ug/ml.

The index of Fractioned Inhibitory Concentration (FIC) is used as a measure of synergy. Double and triple synergy, respectively, were found for the following combinations:
Binary combination Cef + Peptide 19-2.5 : FIC 0,375; binary combination Cef + Peptide 19-4LF : FIC 0.5; binary combination Peptide 19-4LF + Peptide 19-2.5 : FIC 0.5; and ternary combination Cef + Peptide 19-2.5 + Peptide 19-4LF : FIC 0.375.

### Example 2

### Synergistic effects on growth of MRSA

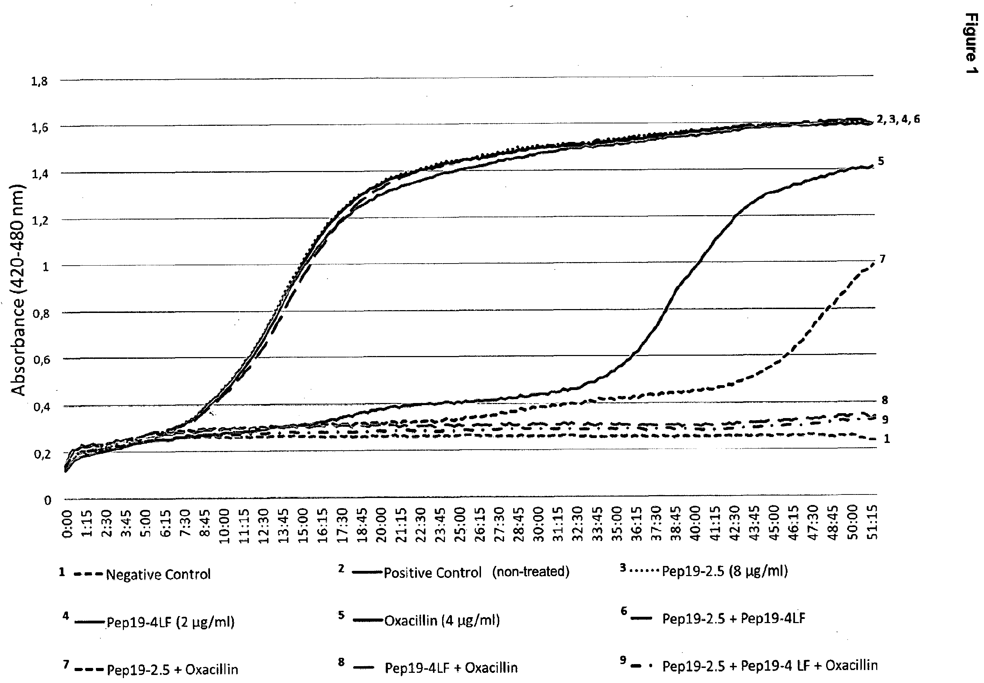

Growth curves of Methicillin Resistant *Staphylococcus aureus* ATCC 43300 (MRSA) exposed at time 0 to combinations of Pep 19-2.5 (8 µg/mL), Pep 19-4LF (2 µg/mL) and oxacillin (4 µg/mL) are shown in Figure 1.

The inhibitory activity of combinations were determined by an automated turbidimetric- based system (Bioscreen C, Labsystem, Helsinki, Finland), which measures absorbance of the culture at regular intervals. Assays were performed in MH broth using Bioscreen polystyrene honeycomb 100-well plates. Inocula consisted of 1 × 10⁵ CFU/mL, approximately in Mueller-Hinton (MH) medium (Difco Laboratories, Sparks, MD, USA). Cell suspensions were grown at 37°C with shaking (control set at "medium r.p.m." position) and the absorbance was determined every 15 min for at least 48 h.

The absorbance of the cultures was measured every 15 minutes using an automated Bioscreen C system.

### Example 3

### Inactivation of biofilm

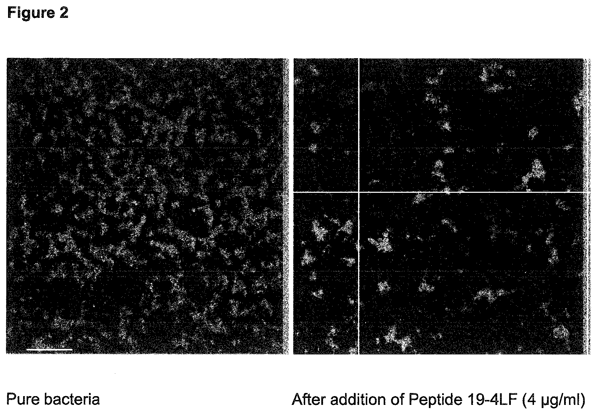

The data shown in Figure 2 indicate that already after short-time treatment (1 h) most bacteria are inactivated. The representative data shown in Figure 2 indicated that after short-time of Peptide 19-4LF addition (1h) the most bacteria are inactivated. The micrographics shown that only a small fraction of the surface was covered by live bacteria forming a biofilm after 1h (B) compared with the untreated one (A). The degree of reduction in life bacteria was very obvious, suggesting the Peptide 19-4LF can penetrate the extracellular polymeric substance (EPS) matrix and kill the bacteria after a short time period.

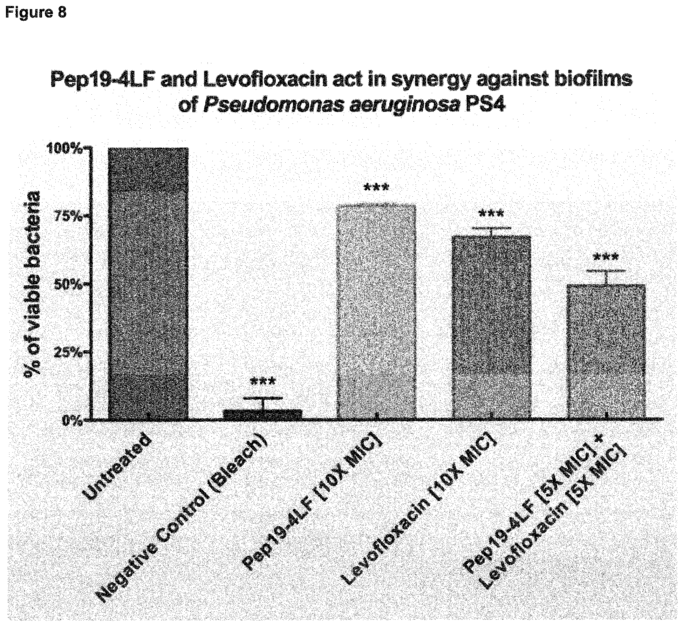

The data shown in Figure 8 were obtained in experiments carried out using the Center for Disease Control (CDC) Biofilm Reactor (CBR). In this case, clinical strain *Pseudomonas aeruginosa* PS4 was incubated in TSB under continuous shaking for 24 hours, followed by additional 24 hours growth with a flow of diluted TSB. Samples received treatments during another 24 hours dissolved at Phosphate Buffer at 37°C. Biofilms were scraped and serially diluted prior to plating and counting. For confocal microscopy, Biofilms were stained using Live/Death BacLight (Life technologies, Carlsbad, California, USA). Results show the average of two independent experiments.

### Example 4

### Kinetics of inhibitory synergistic action: antibiotic peptides of the invention in combination with levofloxacin

Combinations of Peptide 19-2.5 and Peptide 19-4LF with the antibiotic levofloxacin (third generation drug from the group of fluorochinolones) and combinations of Peptide 19-2.5 and Peptide 19-4LF alone have been tested on multiresistant bacteria from *Pseudomonas aeruginosa* PS4.

In particular, the inhibitory activity of combinations were determined by an automated turbidimetric- based system (Bioscreen C, Labsystem, Helsinki, Finland), which measures absorbance of the culture at regular intervals. Assays were performed in MH broth using Bioscreen polystyrene honeycomb 100-well plates. Inocula consisted of 1 × 10⁵ CFU/mL, approximately in Mueller-Hinton (MH) medium (Difco Laboratories, Sparks, MD, USA). Cell suspensions were grown at 37°C with shaking (control set at "medium r.p.m." position) and the absorbance was determined every 15 min for at least 48 h. Each experiment was repeated three times independently and the results were analyzed with the Prism program. For this purpose, first the area under the curve was obtained for each triplicate and the average result was statistically analyzed using the nonparametric Mann-Whitney U supplemented with Kruskal Wallis test for pairwise comparisons.

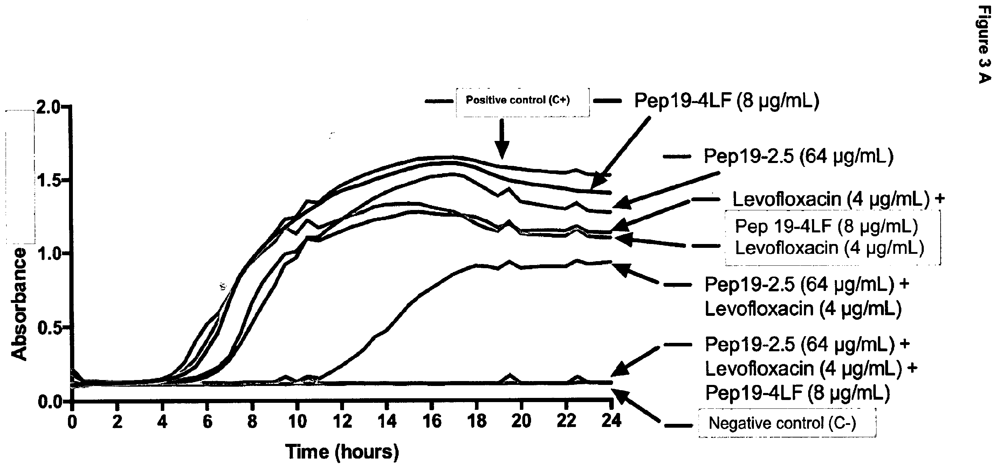

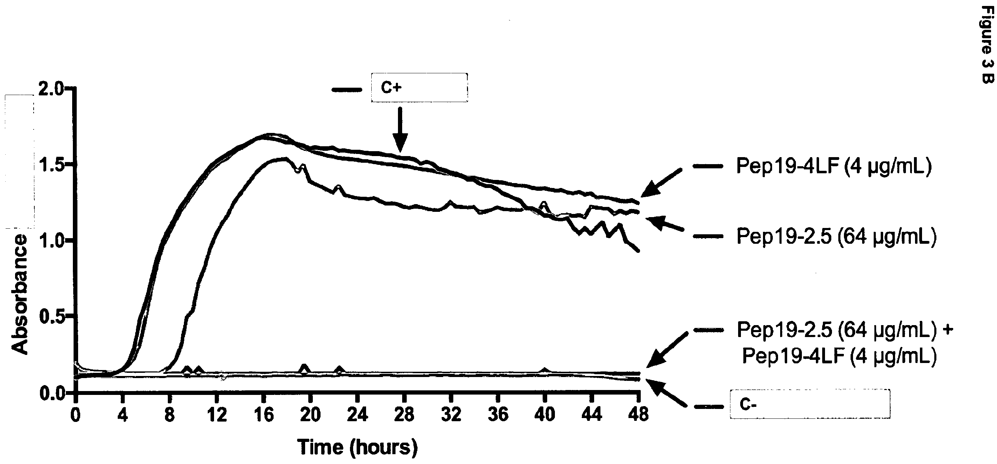

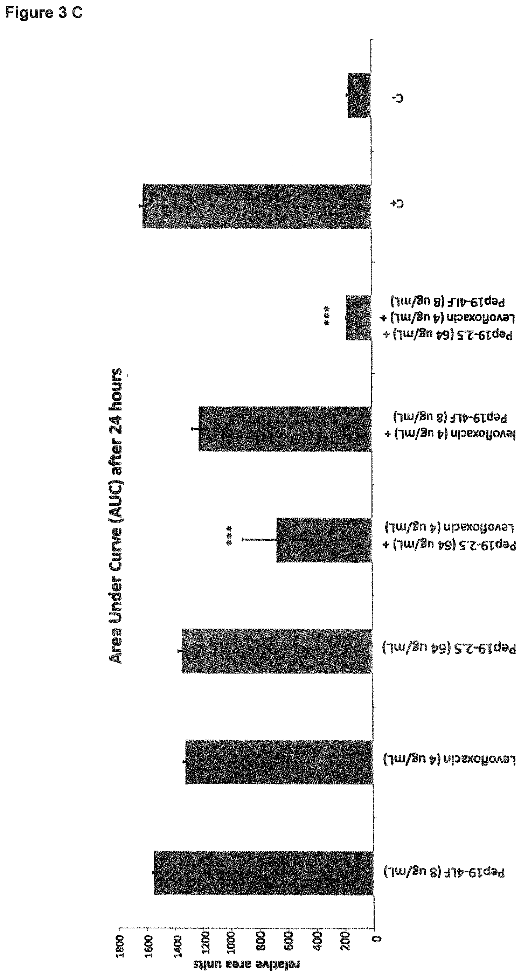

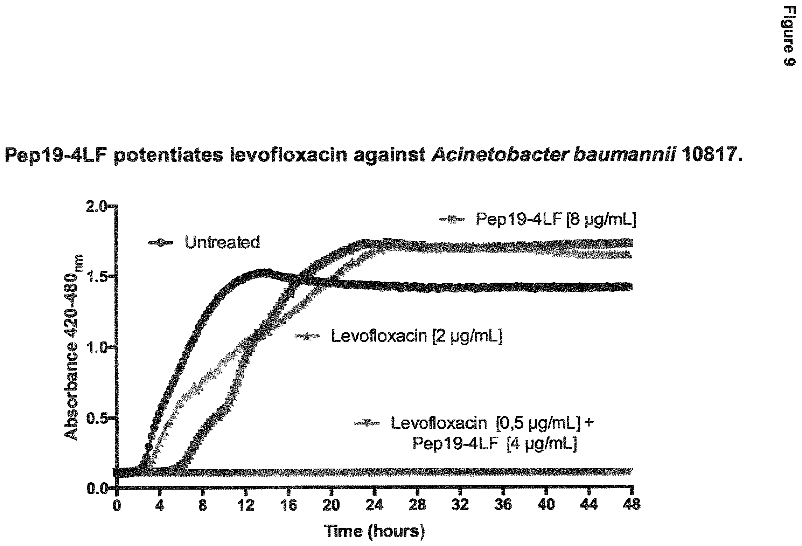

In Figures 3 and 9, the growth of the bacteria (measured optically: optical density) is plotted versus time (in hours). Both combinations, the peptides with the antibiotic as well as the peptides alone are potent synergistic combinations, evidenced by FIC values below 0.5 (FIC=0.31 in Figure 9).

The peptides alone as well as with antibiotics (beside levofloxacin also gentamycin) act synergistically against multi-resistant strains from *Pseudomonas aeruginosa* as well as from *Acinetobacter baumanii.*

Kinetics of inhibitory synergistic action: antibiotic peptides of the invention in combination with gentamycin and oxacillin, respectively.

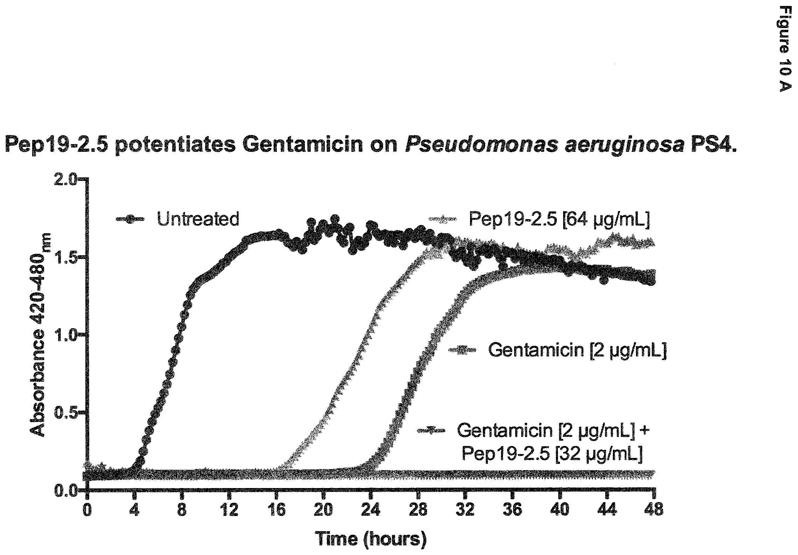

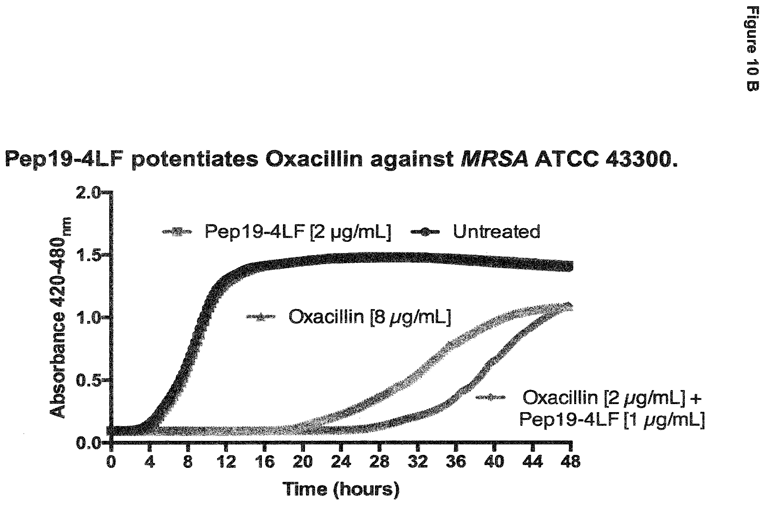

The data shown in Figure 10 were obtained in growth experiments in Mueller-Hinton (cation adjusted) with continuous shaking at 37°C using Bioscreen C, Labsystem, Helsinki, Finland. Concentrations were adjusted after checkerboard analysis, once Fractional Inhibitory Concentrations (FIC) were calculated. All cases resulted in a FIC index <0,5, indicating synergistic effect. Results show the average of three independent experiments.

### Example 5

### Healing attempt according to the German Arzneimittelgesetz §4b with a non-approved drug.

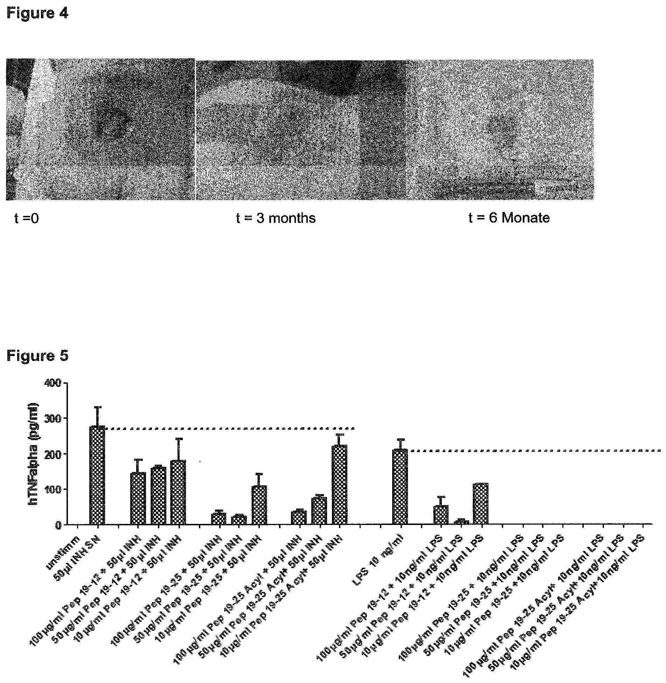

A male patient (78 years old) had - due to a cured tumor in the back - an open wound (see picture at t = 0). Because of the bad soft tissue conditions after radiotherapy, it was decided to perform an operative rehabilitation which, however, did not succeed. Therefore, a conservative wound treatment was initiated. The wound's localization required the involvement of an ambulant nursing service who took over the daily care. Nevertheless, in sporadic intervals, wound infections occurred which inhibited the healing process. A microbiological analysis showed the occurrence of *Staphylococcus aureus, Parvimonas micra,* ß-hemolysing *Streptococcus,* and *Pseudomonas aeruginosa.* Over 6 years, all therapeutical approaches with different antibiotics and topically applied salve formulations failed. In November 2014 the patient was informed about the possibility of a healing attempt', to which he agreed. The therapy was started with 0.1% Pep19-2.5 (Aspidasept^{®}) in salve (BACHEM Lot. 1053821 in DAC-base cream from pharmacist), which showed no effect. Only after increase of the concentration to 1% a significant effect was observed (see picture below at t = 3 months in February 2015), connected with an increasing healing of the wound. Already after 2 months the diameter of the wound was reduced by 50%, and completely healed after 6 months; see Figure 4. This therapeutically effect can be explained only from the use of the Aspidasept^{®} salve, since all other parameters were not changed. The wound is now completely closed.

### Example 6

### Inhibition of the inflammatory response induced by cell-wall compounds of Mycobacterium tuberculosis

*M. tuberculosis* bacteria were treated with the anti-Mtb first line antibiotics isoniazid (INH) or rifampicin (RIF) for 3 days at 37°C. Subsequently, the supernatant of the bacterial cells was added to human mononuclear cells (5 x 10⁵ cells/mi) in the absence or presence of the peptides Pep19-2.5, Pep19-12 and Pep19-2.5 Acyl (Hexanoic residue). The inflammatory response was monitored by measuring tumor-necrosis-factor α (TNFα) formation in an ELISA. It was observed that compound Pep19-2.5 exhibits the strongest anti-TNFα activity, already at a rather low concentration of 10 µg/ml the TNFα formation was inhibited (Figure on the left) by more than 50%. Other peptides with sequence variations showed a weaker inhibitory activity. As control, the inactivation of the LPS-induced TNFα production is presented for the three investigated peptides on the right side of the figure, with a very efficient inhibition as previously described by T. Gutsmann et al. (Gutsmann et al., New antiseptic peptides to protect against endotoxin-mediated shock, AAC 54, 3817-3824 (2010)). See Figure 5.

### Example 7

### Aspidasept^{®} inhibits maturation and migration of MoDCs

Maturation and migration of immature dendritic cells are key steps in the initiation of adaptive immunity against pathogens. However, sustained and excessive inflammatory responses mediated by activated T cells may contribute to chronic inflammation and delayed wound healing.

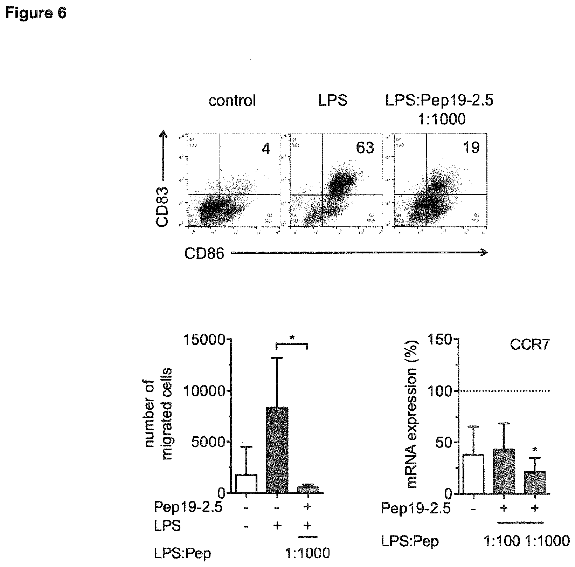

In monocyte-derived dendritic cells (MoDCs), Pep19-2.5 inhibited LPS-mediated upregulation of the maturation marker CD83 and the co-stimulatory molecule CD80 at a peptide:LPS weight ratio of 1000:1. In addition, LPS-induced migration of MoDCs and CCR7 gene expression was completely blocked by Pep19-2.5; see Figure 6.

### Example 8

### Aspisasept^{®} and Aspisasept II reduce IL-8 release in TLR2/6-activated keratinocytes

It can be shown that the keratinocytes from human skin do not react to Gram-negative endotoxin (LPS), apparently due to the lack of the TLR4-receptor and the fact that most bacteria from the skin are of Gram-positive origin. Thus, the effect of Pep19-2.5 and Pep19-4LF on the toxin (pathogenicity factor) FSL-1 (fibroblast-stimulating lipopeptide), a TLR-2/6 activating compound, was checked.

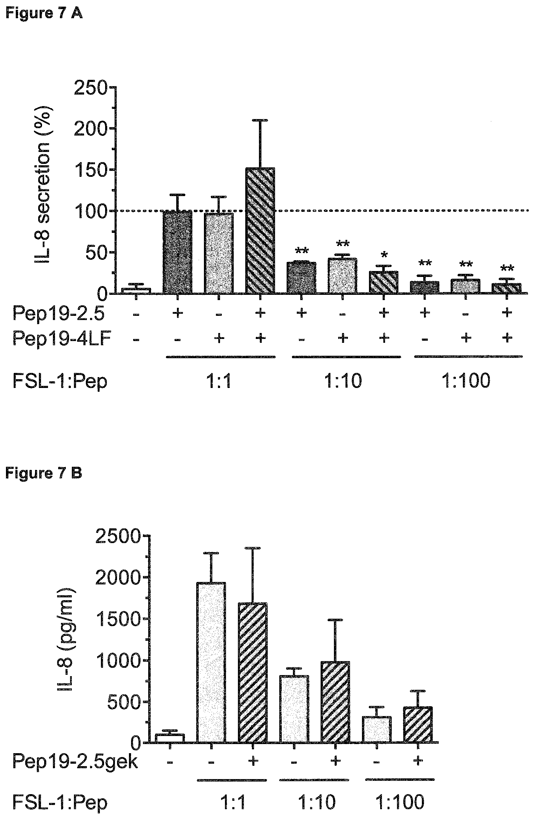

It could be shown that the IL-8 inducing activity of FSL-1 was considerably reduced by the addition of the peptides already at a 10:1 weight ratio, whereas the control peptide Pep19-2.5gek, lacking the C-terminal end of the two peptides, was completely inactive; see Figure 7.

### Example 9

### Suppression of inflammatory responses in skin cells and promotion of keratinocyte migration

The potential of Peptide 19-2.5 and the structurally related compound Peptide 19-4LF has been investigated for their therapeutic application in bacterial skin infections. Primary human keratinocytes responded to TLR2 (FSL-1) but not TLR4 (LPS) activation by increased IL-8 production which was determined with ELISA. Both SALPs inhibited FSL-1-induced phosphorylation of NF-κB p65 and p38 MAPK and significantly reduced IL-8 release and gene expression of IL-1β, CCL2 (MCP-1) and hBD-2. To detect phosphorylation of the intracellular proteins Western Blot was performed. Gene expression was evaluated by quantitative real-time PCR. In the MTT test cytotoxicity was observed at SALP concentrations below 10 µg/ml. In LPS-stimulated monocyte-derived dendritic cells, the peptides blocked IL-6 secretion, downregulated expression of the maturation markers CD83 and CD86 - detected with flow cytometry - and inhibited CCR7-dependent migration capacity. Similarly, monocyte-derived Langerhans-like cells activated with LPS and pro-inflammatory cytokines showed reduced IL-6 levels and CD83/CD86 expression in the presence of SALPs. In addition to acute inflammation, bacterial infections often result in impaired wound healing. Since re-epithelialization is a critical step in wound repair, we tested whether Peptide 19-2.5 affects keratinocyte migration. In a scratch assay the peptide markedly promoted cell migration and accelerated artificial wound closure at concentrations as low as 1 ng/ml and was equipotent to TGF-β.

### Example 10

### Synergistic action of two peptides of the invention in further bacteria

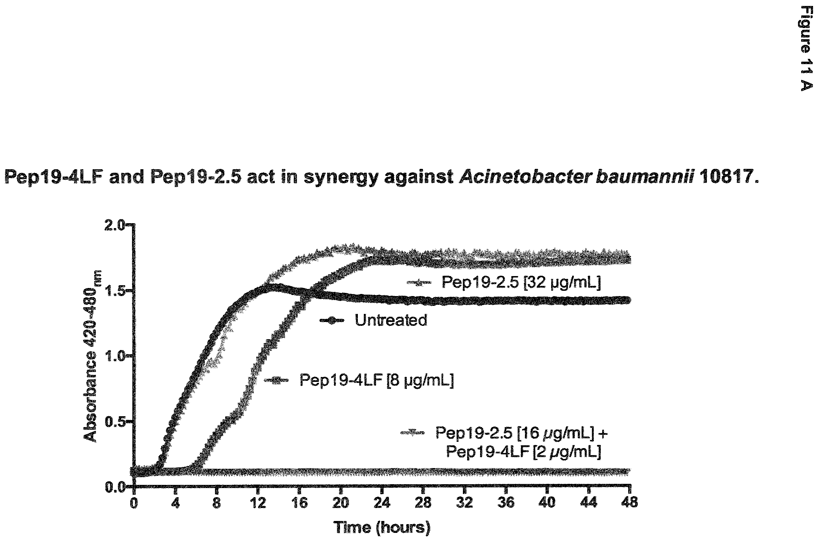

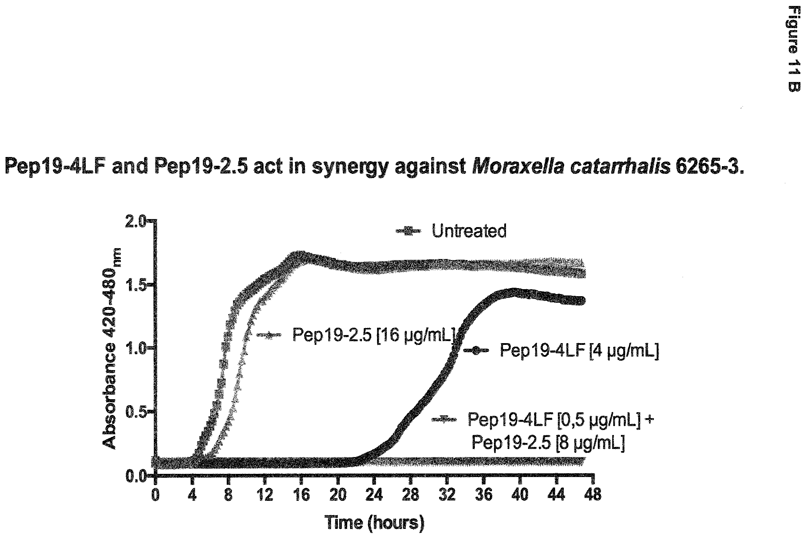

Figure 11 shows data obtained in growth experiments in Mueller-Hinton (cation adjusted) with continuous shaking at 37°C using Bioscreen C, Labsystem, Helsinki, Finland. Concentrations were adjusted after checkerboard analysis, once Fractional Inhibitory Concentrations (FIC) were calculated. All cases resulted in a FIC index <0,5, indicating synergistic effect. Results show the average of three independent experiments.

## Claims

1. A peptide consisting of or comprising 18 to 22 amino acids, wherein the amino acids in positions 1 to 22, counted from the N-terminus, are as follows (1) G; (2) C or lacking; (3) K; (4) K; (5) Y or F; (6) R; (7) R; (8) F; (9) K or R; (10) W; (11) K; (12) F; (13) K or R; (14) G; (15) K; (16) F, L or W; (17) F or W; (18) F, L or W; (19) W or F; (20) C or lacking; (21) F or G or lacking; (22) G or lacking for use in a method of
(a) treating, ameliorating or preventing a bacterial infection of the skin;
(b) treating, ameliorating or preventing a bacterial wound infection; and/or
(c) promoting wound healing.

2. The peptide for use of claim 1, wherein
(a) said peptide
(i) comprises or consists of the sequence of SEQ ID NO: 1.or
(ii) comprises or consists of the sequence of SEQ ID NO:2; and/or
(b) said use is topical.

3. The peptide for use of claim 1 or 2, wherein said bacterial infection of the skin or said bacterial wound infection is an infection by Gram positive and/or Gram negative bacteria, preferably by one or more of Staphylococcus such as Staphylococcus aureus including MRSA, β-hemolytic Streptococcus including group A Streptococcus such as Streptococcus pyogenes, Parvimonas including Parvimonas micra, Pseudomonas such as Pseudomonas aeruginosa including MDRPA, Haemophilus including Haemophilus influenza, Clostridium including Clostridium difficile, Enterococcus including vancomycin-resistant Enterococcus (VRE), Porphyromonas including Porphyromonas gingivalis, Propionibacterium including Propionibacterium acne and Acinetobacter including Acinetobacter baumannii.

4. The peptide for use of any one of claims 1 to 3, wherein said bacterial infection of the skin or said bacterial wound infection is selected from erysipelas, impetigo, folliculitis, boil, cellulitis and carbuncle.

5. A pharmaceutical composition comprising or consisting of a combination of
(a) two or more peptides, each peptide consisting of or comprising 17 to 23 amino acids, wherein the amino acids in positions 1 to 23, counted from the N-terminus, are as follows (1) G, S or lacking; (2) C or lacking; (3) K or R; (4) K or R; (5) Y, W or F; (6) K or R; (7) K or R; (8) F, W or L; (9) K or R; (10) K or L or lacking; (11) W, L or F; (12) K or R; (13) F, Y or C; (14) K or R; (15) G or Q; (16) K or R; (17) F, L or W; (18) F or W; (19) F, L or W; (20) W or F; (21) C or lacking; (22) F or G or lacking; (23) G or lacking; or
(b) one or more peptides, each peptide consisting of or comprising 17 to 23 amino acids, wherein the amino acids in positions 1 to 23, counted from the N-terminus, are as follows (1) G, S or lacking; (2) C or lacking; (3) K or R; (4) K or R; (5) Y, W or F; (6) K or R; (7) K or R; (8) F, W or L; (9) K or R; (10) K or L or lacking; (11) W, L or F; (12) K or R; (13) F, Y or C; (14) K or R; (15) G or Q; (16) K or R; (17) F, L or W; (18) F or W; (19) F, L or W; (20) W or F; (21) C or lacking; (22) F or G or lacking; (23) G or lacking
and one or more antibiotics selected from small organic molecule antibiotics such as ceftriaxone, oxacillin, amoxicillin, amikacin, ciprofloxacin, erythromycin, imipenem and tetracycline, and peptidic antibiotics such as daptomycin and vancomycin;
wherein said combination is synergistic, and wherein synergism occurs with regard to antibacterial activity.

6. The pharmaceutical composition of claim 5, wherein said pharmaceutical composition is a broad-spectrum antibiotic.

7. The pharmaceutical composition of claim 5 or 6, wherein said combination is
(a) a binary combination of
(i) a peptide comprising or consisting of the sequence of SEQ ID NO: 1 and a peptide comprising or consisting of the sequence of SEQ ID NO: 2;
(ii) a peptide comprising or consisting of the sequence of SEQ ID NO: 1 and an antibiotic as defined in claim 6; or
(iii) a peptide comprising or consisting of the sequence of SEQ ID NO: 2 and an antibiotic as defined in claim 6;
or
(b) a ternary combination of a peptide comprising or consisting of the sequence of SEQ ID NO: 1, a peptide comprising or consisting of the sequence of SEQ ID NO: 2 and an antibiotic as defined in claim 6.

8. A pharmaceutical composition of any of claims 5 to 7 for use in
(a) a method of treating, ameliorating or preventing one or more conditions selected from sepsis, bacterial infections of the respiratory tract, bacterial infections of the gastrointestinal tract, bacterial infections of the urogenital tract, necrotizing fasciitis, bacterial infections of burns, bacterial wound infections, and bacterial infections of the skin; or
(b) a method of promoting wound healing.

9. The pharmaceutical composition for use of claim 8, wherein
(a) the bacterial infection of the respiratory tract is tuberculosis, cystic fibrosis or COPD;
(b) the bacterial infection of the gastrointestinal tract is Morbus Crohn; or
(c) said condition is caused by Gram positive and/or Gram negative bacteria, especially by one or more of Staphylococcus such as Staphylococcus aureus including MRSA, Mycobacterium such as Mycobacterium tuberculosis including MDR and XDR strains, Pseudomonas such as Pseudomonas aeruginosa including MDRPA, Enterococcus including vancomycin-resistant Enterococcus (VRE), Haemophilus including Haemophilus influenzae, E. coli including ESBL, Klebsiella including Klebsiella pneumonia, β-hemolytic Streptococcus including group A Streptococcus such as Streptococcus pyogenes, and Acinetobacter including Acinetobacter baumannii.

10. Use of a pharmaceutical composition of any one of claims 5 to 7 or a peptide as defined in any one of claims 1 or 2 for preventing or reducing formation of a biofilm on a device for intra-corporeal use or on a surface in a hospital and/or for removing of a biofilm from a device for intra-corporeal use or surface in a hospital, wherein said device is not present in a human or animal body.

## Patentansprüche

1. Peptid, bestehend aus oder umfassend 18 bis 22 Aminosäuren, wobei die Aminosäuren in den Positionen 1 bis 22, gezählt vom N-Terminus, wie folgt sind: (1) G; (2) C oder fehlend; (3) K; (4) K; (5) Y oder F; (6) R; (7) R; (8) F; (9) K oder R; (10) W; (11) K; (12) F; (13) K oder R; (14) G; (15) K; (16) F, L oder W; (17) F oder W; (18) F, L oder W; (19) W oder F; (20) C oder fehlend; (21) F oder G oder fehlend; (22) G oder fehlend,
zur Verwendung in einem Verfahren zur
(a) Behandlung, Linderung oder Vorbeugung einer bakteriellen Infektion der Haut;
(b) Behandlung, Linderung oder Vorbeugung einer bakteriellen Wundinfektion; und/oder
(c) Förderung der Wundheilung.

2. Peptid zur Verwendung nach Anspruch 1, wobei
(a) das besagte Peptid
(i) die Sequenz SEQ ID NO: 1 umfasst oder aus ihr besteht, oder
(ii) die Sequenz SEQ ID NO: 2 umfasst oder aus ihr besteht; und/oder
(b) diese Verwendung topisch erfolgt.

3. Peptid zur Verwendung nach Anspruch 1 oder 2, wobei die bakterielle Infektion der Haut oder die bakterielle Wundinfektion eine Infektion durch Gram-positive und/oder Gram-negative Bakterien ist, vorzugsweise durch einen oder mehrere von Staphylococcus wie Staphylococcus aureus einschließlich MRSA, β-hemolytische Streptococcus einschließlich Streptococcus der Gruppe A wie Streptococcus pyogenes, Parvimonas, einschließlich Parvimonas micra, Pseudomonas wie Pseudomonas aeruginosa einschließlich MDRPA, Haemophilus einschließlich Haemophilus influenza, Clostridium einschließlich Clostridium difficile, Enterococcus einschließlich Vancomycin-resistente Enterococcus (VRE), Porphyromonas einschließlich Porphyromonas gingivalis, Propionibacterium einschließlich Propionibacterium acne und Acinetobacter einschließlich Acinetobacter baumannii.

4. Peptid zur Verwendung nach einem der Ansprüche 1 bis 3, wobei die bakterielle Infektion der Haut oder die bakterielle Wundinfektion ausgewählt ist aus Erysipel, Impetigo, Follikulitis, Furunkel, Cellulitis und Karbunkel.

5. Pharmazeutische Zusammensetzung, umfassend oder bestehend aus einer Kombination von
(a) zwei oder mehr Peptide, wobei jedes Peptid aus 17 bis 23 Aminosäuren besteht oder diese umfasst, wobei die Aminosäuren in den Positionen 1 bis 23, gezählt vom N-Terminus, wie folgt sind: (1) G, S oder fehlend; (2) C oder fehlend; (3) K oder R; (4) K oder R; (5) Y, W oder F; (6) K oder R; (7) K oder R; (8) F, W oder L; (9) K oder R; (10) K oder L oder fehlend; (11) W, L oder F; (12) K oder R; (13) F, Y oder C; (14) K oder R; (15) G oder Q; (16) K oder R; (17) F, L oder W; (18) F oder W; (19) F, L oder W; (20) W oder F; (21) C oder fehlend; (22) F oder G oder fehlend; (23) G oder fehlend; oder
(b) ein oder mehrere Peptide, wobei jedes Peptid aus 17 bis 23 Aminosäuren besteht oder diese umfasst, wobei die Aminosäuren in den Positionen 1 bis 23, gezählt vom N-Terminus, wie folgt sind: (1) G, S oder fehlend; (2) C oder fehlend; (3) K oder R; (4) K oder R; (5) Y, W oder F; (6) K oder R; (7) K oder R; (8) F, W oder L; (9) K oder R; (10) K oder L oder fehlend; (11) W, L oder F; (12) K oder R; (13) F, Y oder C; (14) K oder R; (15) G oder Q; (16) K oder R; (17) F, L oder W; (18) F oder W; (19) F, L oder W; (20) W oder F; (21) C oder fehlend; (22) F oder G oder fehlend; (23) G oder fehlend
und ein oder mehrere Antibiotika, ausgewählt aus kleinen organischen Molekülen wie Ceftriaxon, Oxacillin, Amoxicillin, Amikacin, Ciprofloxacin, Erythromycin, Imipenem und Tetracyclin, und peptidischen Antibiotika wie Daptomycin und Vancomycin;
wobei diese Kombination synergistisch ist und Synergismus in Bezug auf die antibakterielle Aktivität auftritt.

6. Pharmazeutische Zusammensetzung nach Anspruch 5, wobei die pharmazeutische Zusammensetzung ein Breitspektrum-Antibiotikum ist.

7. Pharmazeutische Zusammensetzung nach Anspruch 5 oder 6, wobei die Kombination
(a) eine binäre Kombination ist aus
(i) einem Peptid, das die Sequenz von SEQ ID NO: 1 umfasst oder aus dieser besteht, und einem Peptid, das die Sequenz von SEQ ID NO: 2 umfasst oder aus dieser besteht;
(ii) einem Peptid, das die Sequenz von SEQ ID NO: 1 umfasst oder aus dieser besteht, und einem wie in Anspruch 6 definierten Antibiotikum; oder
(iii) einem Peptid, das die Sequenz von SEQ ID NO: 2 umfasst oder aus dieser besteht, und einem wie in Anspruch 6 definierten Antibiotikum;
oder
(b) eine ternäre Kombination ist aus einem Peptid, das die Sequenz von SEQ ID NO: 1 umfasst oder aus dieser besteht, einem Peptid, das die Sequenz von SEQ ID NO: 2 umfasst oder aus dieser besteht, und einem wie in Anspruch 6 definierten Antibiotikum.

8. Pharmazeutische Zusammensetzung nach einem der Ansprüche 5 bis 7 zur Verwendung in
(a) einem Verfahren zur Behandlung, Linderung oder Vorbeugung eines oder mehrerer Leiden, ausgewählt aus Sepsis, bakterieller Infektionen des Respirationstrakts, bakterieller Infektionen des Gastrointestinaltrakts, bakterieller Infektionen des Urogenitaltrakts, nekrotisierender Fasziitis, bakterieller Infektionen von Verbrennungen, bakterieller Wundinfektionen und bakterieller Infektionen Haut; oder
(b) einer Methode zur Förderung der Wundheilung.

9. Pharmazeutische Zusammensetzung zur Verwendung nach Anspruch 8, wobei
(a) die bakterielle Infektion der Atemwege Tuberkulose, Mukoviszidose oder COPD ist;
(b) die bakterielle Infektion des Magen-Darm-Trakts Morbus Crohn ist; oder
(c) das Leiden durch Gram-positive und/oder Gram-negative Bakterien verursacht wird, insbesondere durch einen oder mehrere von Staphylococcus wie Staphylococcus aureus einschließlich MRSA, Mycobacterium wie Mycobacterium tuberculosis einschließlich MDR- und XDR-Stämmen, Pseudomonas wie Pseudomonas aeruginosa einschließlich MDRPA, Enterococcus einschließlich Vancomycinresistenten Enterococcus (VRE), Haemophilus einschließlich Haemophilus influenzae, E. coli einschließlich ESBL, Klebsiella einschließlich Klebsiella pneumonia, β-hämolytische Streptokokken einschließlich Streptokokken der Gruppe A wie Streptococcus pyogenes, und Acinetobacter, einschließlich Acinetobacter baumannii.

10. Verwendung einer pharmazeutischen Zusammensetzung nach einem der Ansprüche 5 bis 7 oder eines Peptids nach einem der Ansprüche 1 oder 2 zur Verhinderung oder Verringerung der Bildung eines Biofilms auf einer Vorrichtung zur intrakorporalen Anwendung oder auf einer Oberfläche in einem Krankenhaus und/oder zur Entfernung eines Biofilms von einer Vorrichtung zur intrakorporalen Anwendung oder einer Oberfläche in einem Krankenhaus, wobei sich die Vorrichtung nicht in einem menschlichen oder tierischen Körper befindet.

## Revendications

1. Peptide consistant en ou comprenant 18 à 22 acides aminés, dans lequel les acides aminés aux positions 1 à 22, comptées à partir de l'extrémité N-terminale, sont les suivants (1) G ; (2) C ou manquant ; (3) K ; (4) K ; (5) Y ou F ; (6) R ; (7) R ; (8) F ; (9) K ou R ; (10) W ; (11) K ; (12) F ; (13) K ou R ; (14) G ; (15) K ; (16) F, L ou W ; (17) F ou W ; (18) F, L ou W ; (19) W ou F ; (20) C ou manquant ; (21) F ou G ou manquant ; (22) G ou manquant, pour une utilisation dans une méthode de
(a) traitement, amélioration ou prévention d'une infection bactérienne de la peau ;
(b) traitement, amélioration ou prévention d'une infection bactérienne d'une plaie ; et/ou
(c) promotion de la cicatrisation.

2. Peptide pour une utilisation selon la revendication 1,
(a) lequel peptide
(i) comprend ou consiste en la séquence de la SEQ ID NO : 1 ou
(ii) comprend ou consiste en la séquence de la SEQ ID NO : 2 ; et/ou
(b) dans lequel ladite utilisation est topique.

3. Peptide pour une utilisation selon la revendication 1 ou 2, dans lequel ladite infection bactérienne de la peau ou ladite infection bactérienne d'une plaie est une infection par des bactéries Gram positives et/ou Gram négatives, de préférence par un ou plusieurs parmi Staphylococcus tel que Staphylococcus aureus y compris SARM, Streptococcus β-hémolytique y compris Streptococcus du groupe A tel que Streptococcus pyogenes, Parvimonas y compris Parvimonas micra, Pseudomonas telle que Pseudomonas aeruginosa y compris MDRPA, Haemophilus y compris Haemophilus influenza, Clostridium y compris Clostridium difficile, Enterococcus y compris Enterococcus résistant à la vancomycine (ERV), Porphyromonas y compris Porphyromonas gingivalis, Propionibacterium y compris Propionibacterium acne et Acinetobacter y compris Acinetobacter baumannii.

4. Peptide pour une utilisation selon l'une quelconque des revendications 1 à 3, dans lequel ladite infection bactérienne de la peau ou ladite infection bactérienne d'une plaie est choisie parmi un érysipèle, un impétigo, une folliculite, un furoncle, une cellulite et un anthrax.

5. Composition pharmaceutique comprenant ou consistant en une combinaison de
(a) deux peptides ou plus, chaque peptide consistant en ou comprenant 17 à 23 acides aminés, dans lesquels les acides aminés aux positions 1 à 23, comptées à partir de l'extrémité N-terminale, sont les suivants (1) G, S ou manquant ; (2) C ou manquant ; (3) K ou R ; (4) K ou R ; (5) Y, W ou F ; (6) K ou R ; (7) K ou R ; (8) F, W ou L ; (9) K ou R ; (10) K ou L ou manquant; (11) W, L ou F ; (12) K ou R; (13) F, Y ou C ; (14) K ou R ; (15) G ou Q ; (16) K ou R; (17) F, L ou W ; (18) F ou W ; (19) F, L ou W ; (20) W ou F ; (21) C ou manquant ; (22) F ou G ou manquant ; (23) G ou manquant ; ou
(b) un ou plusieurs peptides, chaque peptide consistant en ou comprenant 17 à 23 acides aminés, dans lesquels les acides aminés aux positions 1 à 23, comptées à partir de l'extrémité N-terminale, sont les suivants (1) G, S ou manquant ; (2) C ou manquant ; (3) K ou R ; (4) K ou R; (5) Y, W ou F ; (6) K ou R; (7) K ou R; (8) F, W ou L ; (9) K ou R; (10) K ou L ou manquant; (11) W, L ou F ; (12) K ou R; (13) F, Y ou C ; (14) K ou R; (15) G ou Q ; (16) K ou R ; (17) F, L ou W ; (18) F ou W ; (19) F, L ou W ; (20) W ou F ; (21) C ou manquant ; (22) F ou G ou manquant ; (23) G ou manquant
et un ou plusieurs antibiotiques choisis parmi les antibiotiques à petites molécules organiques tels que la ceftriaxone, l'oxacilline, l'amoxicilline, l'amikacine, la ciprofloxacine, l'érythromycine, l'imipenème et la tétracycline, et les antibiotiques peptidiques tels que la daptomycine et la vancomycine ;
dans laquelle ladite combinaison est synergique, et dans laquelle la synergie a lieu par rapport à l'activité antibactérienne.

6. Composition pharmaceutique selon la revendication 5, laquelle composition pharmaceutique est un antibiotique à large spectre.

7. Composition pharmaceutique selon la revendication 5 ou 6, dans laquelle ladite combinaison est
(a) une combinaison binaire de
(i) un peptide comprenant ou consistant en la séquence de la SEQ ID NO : 1 et un peptide comprenant ou consistant en la séquence de la SEQ ID NO : 2 ;
(ii) un peptide comprenant ou consistant en la séquence de la SEQ ID NO : 1 et un antibiotique tel que défini dans la revendication 6 ; ou
(iii) un peptide comprenant ou consistant en la séquence de la SEQ ID NO : 2 et un antibiotique tel que défini dans la revendication 6 ; ou
(b) une combinaison ternaire d'un peptide comprenant ou consistant en la séquence de la SEQ ID NO : 1, d'un peptide comprenant ou consistant en la séquence de la SEQ ID NO : 2, et d'un antibiotique tel que défini dans la revendication 6.

8. Composition pharmaceutique selon l'une quelconque des revendications 5 à 7, pour une utilisation dans
(a) une méthode de traitement, d'amélioration ou de prévention d'un ou plusieurs états choisis parmi un sepsis, des infections bactériennes des voies respiratoires, des infections bactériennes des voies gastro-intestinales, des infections bactériennes des voies urogénitales, une fasciite nécrosante, des infections bactériennes de brûlures, des infections bactériennes de plaies, et des infections bactériennes de la peau ; ou
(b) une méthode de promotion de la cicatrisation.

9. Composition pharmaceutique pour une utilisation selon la revendication 8, dans laquelle
(a) l'infection bactérienne des voies respiratoires est une tuberculose, une mucoviscidose ou une BPCO ;
(b) l'infection bactérienne des voies gastro-intestinales est une maladie de Crohn ; ou
(c) ledit état est dû à des bactéries Gram positives et/ou Gram négatives, en particulier à un ou plusieurs parmi Staphylococcus tel que Staphylococcus aureus y compris SARM, Mycobacterium tel que Mycobacterium tuberculosis y compris les souches MDR et XDR, Pseudomonas telle que Pseudomonas aeruginosa y compris MDRPA, Enterococcus y compris Enterococcus résistant à la vancomycine (ERV), Haemophilus y compris Haemophilus influenzae, E. coli y compris ESBL, Klebsiella y compris Klebsiella pneumoniae, Streptococcus β-hémolytique y compris Streptococcus du groupe A tel que Streptococcus pyogenes, et Acinetobacter y compris Acinetobacter baumannii.

10. Utilisation d'une composition pharmaceutique de l'une quelconque des revendications 5 à 7 ou d'un peptide tel que défini dans l'une quelconque des revendications 1 ou 2 pour prévenir ou réduire la formation d'un biofilm sur un dispositif destiné à un usage intracorporel ou sur une surface dans un hôpital et/ou pour éliminer un biofilm sur un dispositif destiné à un usage intracorporel ou une surface dans un hôpital, dans laquelle ledit dispositif n'est pas présent dans un corps humain ou animal.
